# EUROPÄISCHE PATENTSCHRIFT

(11) **EP 0 966 508 B1**
(45) Veröffentlichungstag und Bekanntmachung des Hinweises auf die Patenterteilung: **19.02.2003**
(21) Anmeldenummer: 98916941.2
(22) Anmeldetag: 13.03.1998
(51) Int. Cl.: C10G 70/00, C07C 7/00, C07C 11/02

(54) **VERFAHREN ZUR REINIGUNG VON STOFFSTRÖMEN**
PROCESS FOR PURIFYING MATERIAL FLOWS
PROCEDE DE PURIFICATION DE FLUX DE MATIERES

(30) Priorität: 14.03.1997 DE 19710762
(43) Veröffentlichungstag der Anmeldung: 29.12.1999
(73) Patentinhaber: BASF AKTIENGESELLSCHAFT, 67056 Ludwigshafen (DE)
(72) Erfinder: FLICK, Klemens, D-76863 Herxheim (DE); MEISSNER, Ruprecht, D-67273 Weisenheim (DE); ARTRIP, David, J., Prairieville, CA 73070769 (US); KUNZ, Fabian, D-67112 Mutterstadt (DE); HEFNER, Werner, D-68623 Lampertheim (DE); FESER, Rainer, D-67269 Grünstadt (DE)
(86) Internationale Anmeldenummer: EP9801469
(87) Internationale Veröffentlichungsnummer: WO98041597

(56) Entgegenhaltungen:
- EP-A- 0 624 562
- WO-A-92/00261
- US-A- 2 837 587
- US-A- 3 671 603
- US-A- 3 725 377
- US-A- 3 789 581
- US-A- 4 345 105

## Beschreibung

Die vorliegende Erfindung betrifft ein Verfahren zur Entfernung von Verunreinigungen aus Stoffströmen.

In vielen verschiedenen chemischen Verfahren wird ein Stoffstrom an einem oder mehreren Katalysatoren zu Reaktionsprodukten umgesetzt. Ein Beispiel eines solchen Verfahrens ist die Polymerisation von Olefinen wie Ethylen oder Propylen an Polymerisationskatalysatoren zum entsprechenden Polyolefin.

Diesen Verfahren gemeinsam ist die Verwendung eines Katalysators. Katalysatoren sind in den allermeisten Fällen empfindlich gegen bestimmte Verunreinigungen, sogenannte Katalysatorgifte, deren übermäßiger Kontakt mit dem Katalysator diesen in seinen Eigenschaften, wie Aktivität, Selektivität oder Standzeit beeinträchtigt. In der Regel werden durch das Katalysatorgift aktive Zentren des Katalysators belegt und stehen so nicht mehr zur Katalyse der gewünschten Reaktion zur Verfügung. Bei der Verwendung von Katalysatoren ist daher Sorge dafür zu tragen, daß die Einsatzstoffströme, mit denen die Katalysatoren beaufschlagt werden, keine oder höchstens die Menge an Katalysatorgiften mit sich führen, die bei wirtschaftlich vertretbarem Aufwand zur Entfernung von Katalysatorgiften einen wirtschaftlich zufriedenstellenden Betrieb des Katalysators im Hinblick auf Selektivität, Aktivität und Standzeit ermöglichen. Ein bekanntes Katalysatorgift ist beispielsweise Schwefel für viele metallische Katalysatoren.

Manche Verunreinigungen, die in Einsatzstoffströmen enthalten sind, schädigen den verwendeten Katalysator zwar nicht dauerhaft, führen aber zu unerwünschten Nebenreaktionen und vermindern so beispielsweise die Produktqualität. Ein Beispiel für derartige Verunreinigungen sind Alkine in Olefinen bei der katalysierten Olefinpolymerisation, die wichtige Produkteigenschaften wie die Molekulargewichtsverteilung, die Stereospezifität der Polymerisation oder die Stabilität der polymeren Produkte beeinträchtigen. Solche Probleme können selbstverständlich auch bei unkatalysierten Reaktionen auftreten.

In aller Regel durchläuft ein Einsatzstoffstrom daher vor seinem Einsatz in einer Reaktion ein Reinigungsverfahren zur Abtrennung von Verunreinigungen, die in der betreffenden Reaktion stören würden. Es sind zahlreiche derartige Reinigungsverfahren bekannt, beispielsweise Gaswäscheverfahren mit verschiedenen Lösungsmitteln, Verfahren zur Absorption von Verunreinigungen an Absorbentien wie etwa Zeolithen oder Aktivkohlen, oder Verfahren, in denen störende Verunreinigungen durch Membranen aus einem Stoffstrom entfernt werden.

US-A 4,861,939 lehrt beispielsweise ein Verfahren zur Entarsenierung von Naphtha, bei dem das Naphtha durch Kontakt mit einem Nickeloxid und Nickel enthaltenden Adsorbens von arsenhaltigen Verbindungen weitgehend befreit wird.

Verfahren, in denen Katalysatoren eingesetzt werden, um Stoffströme zu reinigen, sind seltener und sind meist nur auf die Entfernung weniger spezieller Verunreinigungen in speziellen Stoffströmen beschränkt. Die hydrierende Entschwefelung von Kohlenwasserstoffen an CoO/MoO₃- oder NiO/MoO₃-Katalysatoren bei 350 bis 450°C und anschließende Absorption des entstehenden Schwefelwasserstoffs; die Hoch- oder Tieftemperaturkonvertierung von Kohlenmonoxid mit Wasser zu Kohlendioxid und Wasserstoff an FeO/Cr₂O₃oder CoO/MoO₃-Katalysatoren und die Methanisierung von CO und CO₂ mit Wasserstoff an Nickelkatalysatoren stellen die bekanntesten Verfahren dar, sie sind allgemeines Lehrbuchwissen.

In Oil & Gas Journal, Ausgabe Oktober 1994, Seiten 50 bis 55, ist der sogenannte "Triple P" oder "Propylene Polishing Process" der Fina Research SA, Feluy, Belgien, beschrieben. Mit diesem Verfahren werden schwefelhaltige Verbindungen, Arsan und Stiban, Sauerstoff, CO und CO2 sowie Wasserstoff aus Propylen entfernt, um so hochreines Propylen für die Polymerisation zu erzeugen. Das Verfahren nutzt ein spezielles, nicht im Detail offenbartes Adsorbens zur adsorptiven Entfernung aller Verunreinigungen außer Wasserstoff, wobei dieses Adsorbens zusätzlich als Katalysator der Hydrierung von Propen mit Wasserstoff wirkt und so den Wasserstoff katalytisch entfernt.

In einem Vortrag von D. J. Artrip, C. Herion und R. Meissner auf der Konferenz "MetCon '93", Session Four, in Houston, Texas, USA, wurde am 27.05.1993 ein vierstufiges Verfahren zur Entfernung von verschiedenen Verunreinigungen aus Olefinströmen für Polymerisationsreaktionen offenbart. In diesem Verfahren werden in einem ersten Schritt arsen- und schwefelhaltige Verbindungen an einem aus Kupfer- und Zinkoxiden bestehenden Absorbens absorbiert. In einem zweiten Schritt werden Acetylene und Diene nach Zugabe einer zu ihrer Hydrierung ausreichenden Menge Wasserstoff an einem palladiumhaltigen Katalysator zu Olefinen hydriert. Im dritten Verfahrensschritt wird vorhandener Sauerstoff an einem metallischen Kupferkontakt entfernt. Dieser Verfahrensschritt ist ein gemischter Verfahrensschritt, bei dem Sauerstoff entweder unter Kupferkatalyse mit Rest-Wasserstoff oder vorhandenem Kohlenmonoxid zur Wasser oder Kohlendioxid reagiert, oder vom metallischen Kupfer unter Bildung von Kupferoxid absorbiert wird. Im vierten und letzten Verfahrensschritt wird noch vorhandenes Rest-Kohlenmonoxid an einem Kupferoxidkatalysator zu Kohlendioxid umgewandelt, wobei das Kupferoxid allmählich zu metallischem Kupfer reduziert wird. Im gleichen Verfahrensschritt wird etwaiger Rest-Wasserstoff ebenfalls zu Wasser oxidiert. Der nach diesem vierstufigen Reinigungsverfahren vorhandene Gehalt des Olefins an Wasser und Kohlendioxid kann mit konventionellen Techniken entfernt werden. Dieses vierstufige Verfahren weist den Nachteil auf, daß ein Alkin- oder Diengehalt des Einsatzstoffstroms im ersten Verfahrensschritt zu zunehmenden Ablagerungen in den Apparaten, dem sogenannten "Fouling", das bis zur Blockierung der Apparate gehen kann, führt. Zudem fehlt einem Verfahren, bei dem unter Umständen Acetylen in der ersten Verfahrensstufe mit einem Kupferkatalysator in Kontakt kommt, aufgrund von Sicherheitsbedenken trotz der theoretisch nahezu unmöglichen Bildung des des hochexplosiven Kupferacetylids bisher die Akzeptanz auf dem Markt.

US-A-2,837,587 offenbart ein Verfahren zur Entfernung von Acetylen und Kohlenmonoxid aus Olefinströmen, bei dem in einer ersten Verfahrensstufe Acetylen durch Hydrierung an einem Kupferkatalysator und in einer zweiten Stufe Kohlenmonoxid durch Absorption an Hopcalit, einer Kupfer- und Manganoxide enthaltenden Absorptionsmasse, entfernt wird.

US-A-3,725,377 lehrt ein Verfahren zur Reinigung von 1,3-Butadien, bei dem ein verunreinigter Butadienstrom, der zum Einsatz in der Polymerisation von 1,3,-Butadien bestimmt ist, nacheinander durch Hydrierung von Verunreinigungen, fraktionierte Destillation und Absorption von weiteren Verunreinigungen an Silicagel, Aktivkohle, Aluminiumoxid, natürlichen oder künstlichen Molekularsieben gereinigt wird.

Angesichts der großen Bedeutung von gereinigten Stoffströmen in der industriellen Chemie besteht nach wie vor Bedarf an neuen und verbesserten Gasreinigungsverfahren. Es ist die Aufgabe der vorliegenden Erfindung, ein Reinigungsverfahren zu finden, mit dem möglichst viele typische Verunreinigungen in möglichst wenig Verfahrensstufen aus Stoffströmen entfernt werden können, ohne Nachteile der bekannten Verfahren in Kauf nehmen zu müssen.

Demgemäß wurde ein Verfahren zur Entfernung von Alkinen, Sauerstoff, Verbindungen, die Schwefel, Arsen und/oder Antimon enthalten, wahlweise auch Kohlenmonoxid und wahlweise auch Dienen oder Dienen und einfach ungesättigten Kohlenwasserstoffen aus Stoffströmen, die Alkine, Sauerstoff, Verbindungen, die Schwefel, Arsen und/oder Antimon enthalten, und Kohlenmonoxid umfassen gefunden, das, dadurch gekennzeichnet ist, daß man höchstens drei Verfahrensstufen anwendet, wobei man in der genannten Reihenfolge:
- in einer ersten Verfahrensstufe nach Zugabe von Wasserstoff zum Einsatzstoffstrom an einem Silber und Palladium enthaltenden Hydrierkatalysator mit einem Siliciumdioxid enthaltenden anorganischen oxidischen Träger Alkine, zumindest teilweise auch Sauerstoff, und wahlweise auch Diene oder Diene und einfach ungesättigte Kohlenwasserstoffe durch Hydrierung entfernt,
- restlichen Sauerstoff aus der ersten Verfahrensstufe und Schwefel, Arsen und/oder Antimon enthaltende Verbindungen in einer zweiten Verfahrensstufe an einer Kontaktmasse durch Absorption abreichert oder entfernt und gegebenenfalls restlichen Sauerstoff mit Kohlenmonoxid und/oder restlichem Wasserstoff zumindest teilweise an dieses Kontaktmasse katalytisch zu Wasser und Kohlendioxid umsetzt;
- gegebenenfalls in einer dritten Verfahrensstufe restliches Kohlenmonoxid und/oder restlichen Wasserstoff aus den vorangegangenen Verfahrensstufen an einer Kontaktmasse in Kohlendioxid und/oder Wasser umwandelt.

Das erfindungsgemäße Verfahren ist insbesondere geeignet, Verunreinigungen aus Olefinströmen für die Polymerisation, speziell an Polymerisationskatalysatoren, aus in Kunststoffrecyclinganlagen erzeugten Pyrolysegasen oder aus durch mit Schwefel enthaltenden Verbindungen und durch mit mittels Hydrierung entfernbaren Verbindungen verunreinigten Stoffströmen zu entfernen. Mit diesem Verfahren kann eine vergleichsweise hohe Zahl verschiedener Verunreinigungen aus verschiedenen Stoffströmen in vergleichsweise wenigen Verfahrensstufen entfernt werden, wobei Foulingprobleme vermieden werden und die Bildung von Kupferacetyliden prinzipiell ausgeschlossen ist.

Die erste Verfahrensstufe umfaßt die Hydrierung von Acetylenen, Olefinen und Sauerstoff an einem gegen Schwefel, Arsen und/oder Antimon enthaltenden Verbindungen weitgehend beständigen festen Hydrierkatalysator. Weitgehend beständig bedeutet, daß der Katalysator über wirtschaftlich akzeptable Standzeiten, beispielsweise über einige Monate oder Jahre hinweg betrieben werden kann, wobei während der Gesamtlebensdauer des Katalysator auch Regenerierungsvorgänge, beispielsweise zur Entfernung von auf dem Katalysator abgelagertem Koks, durchgeführt werden können.

Derartige, gegen Schwefel, Arsen und/oder Antimon beständige feste Hydrierkatalysatoren sind beispielsweise Hydrierkatalysatoren auf Basis CoO/MoO₃ oder NiO/MoO₃. Im Verfahren der vorliegenden Erfindung ist die Verwendung dieser Katalysatoren nicht bevorzugt, da sie in der Regel bei Temperaturen deutlich oberhalb von 200°C betrieben werden müssen. Zur Herstellung hochreiner Stoffströme ist es allgemein empfehlenswert, bei möglichst niedrigen Temperaturen zu arbeiten, da bei höheren Temperaturen Nebenreaktionen, die zur Bildung von störenden Komponenten führen können, verstärkt auftreten.

In bevorzugter Weise wird im Verfahren der vorliegenden Erfindung ein mindestens ein Edelmetall enthaltender Hydrierkatalysator für den ersten Verfahrensschritt verwendet. Besonders geeignet sind Katalysatoren, die mindestens ein Metall der 8. Nebengruppe des Periodensystems der Elemente, insbesondere Palladium enthalten. Die Katalysatoren können darüber hinaus noch weitere Elemente oder Elementverbindungen als Promotoren enthalten. Es wird ein Katalysator verwendet, der Palladium, Silber und einen Katalysatorträger auf Siliciumdioxidbasis, insbesondere Kieselgur, umfaßt. Die BET-Oberfläche dieses Trägers beträgt im allgemeinen mindestens 2 m²/g, beispielsweise mehr als 5 m²/g und in bevorzugter Weise mehr als 10 m²/g sowie im allgemeinen höchstens 400 m²/g, beispielsweise weniger als 300 m²/g und in bevorzugter Weise weniger als 200 m²/g.

Besonders geeignet ist ein Katalysator, der mindestens 0,05 Gew.-% Palladium, beispielsweise mehr als 0,1 Gew.-% Palladium und in bevorzugter Weise mehr als 0,2 Gew.-% Palladium und höchstens 1,5 Gew.-% Palladium, beispielsweise weniger als 1,0 Gew.-% Palladium und in bevorzugter Weise weniger als 0,7 Gew.-% Palladium sowie mindestens 0,05 Gew.-% Silber, beispielsweise mehr als 0,1 Gew.-% Silber und in bevorzugter Weise mehr als 0,2 Gew.-% Silber und höchstens 1,5 Gew.-% Silber, beispielsweise weniger als 1,0 Gew.-% Silber und in bevorzugter Weise weniger als 0,7 Gew.-% Silber auf einem Kieselgur-Katalysatorträger mit einer BET-Oberfläche im Bereich von 10 m²/g bis 40 m²/g enthält. Derartige Katalysatoren weisen überraschenderweise eine hohe Toleranz gegenüber Schwefel, Arsen und Antimon enthaltenden Verbindungen auf, die ihren Einsatz zur Hydrierung von Verunreinigungen in Stoffströmen in wirtschaftlich befriedigender Weise ermöglicht.

Verfahren zur Herstellung solcher Katalysatoren sind dem Fachmann bekannt. Beispielsweise können solche Katalysatoren durch Tränkung des Trägers oder Trägervorläufers mit einer Lösung von Salzen der aufzubringenden Metalle, etwa mit einer Lösung der Metallnitrate in Salpetersäure, Trocknung, Kalzination und gegebenenfalls einer Reduktion der Aktivkomponenten und/oder Promotoren zum Metall, die beispielsweise durch Hydrierung im Reaktor durchgeführt werden kann, hergestellt werden.

In der ersten Verfahrensstufe werden Sauerstoff, Alkine und wahlweise Diene und/oder einfach ungesättigte Kohlenwasserstoffe durch Hydrierung entfernt, wobei die Durchführung dieser Hydrierung sowohl in der Gasphase als auch in der Flüssigphase möglich ist. Im letzteren Fall kann sowohl eine Riesel- als auch eine Sumpffahrweise gewählt werden. Zur Hydrierung wird die benötigte Menge Wasserstoff vor dem Hydrierreaktor zugegeben. Die zuzugebende Wasserstoffmenge bemißt sich nach den vorhandenen und zu entfernenden Verunreinigungen, wobei vorteilhafterweise ein Überschuß, beispielsweise ein zwei- bis dreifacher Überschuß an Wasserstoff zugegeben wird. Im allgemeinen wird der Wasserstoff im Hydrierreaktor fast vollständig verbraucht. Alkine und Diene werden, verglichen mit einfach ungesättigten Kohlenwasserstoffen, bevorzugt hydriert, so daß mit dem erfindungsgemäßen Verfahren auch eine Entfernung von Alkinen und Dienen aus Olefinströmen möglich ist. Beispielsweise kann in Ethylenströmen vorhandenes Acetylen oder in Propylenströmen vorhandenes Propin oder Propadien oder in Butadienströmen vorhandenes Vinylacetylen hydrierend entfernt werden. Ein gewisser, jedoch vergleichsweise kleiner Anteil an Alkenhydrierung zu den entsprechenden Alkanen ist jedoch meistens unvermeidbar. In dieser ersten Verfahrensstufe wird gemeinsam mit den organischen ungesättigten Verbindungen nach Maßgabe der zugegebenen Wasserstoffmenge auch Sauerstoff aus dem Stoffstrom entfernt.

Die Hydrierung kann unter üblichen Hydrierbedingungen für edelmetallhaltige Hydrierkatalysatoren durchgeführt werden, beispielsweise bei Raumtemperatur oder Temperaturen oberhalb von Raumtemperatur, bevorzugterweise bei oder oberhalb von 40°C und in besonders bevorzugter Weise bei oder oberhalb von 50°C. Wird die Hydrierung in der Gasphase durchgeführt, so wird üblicherweise bei oder unterhalb einer Temperatur von 200°C gearbeitet, bevorzugterweise bei oder unterhalb von 180°C und in besonders bevorzugter Weise bei oder unterhalb von 150°C. Wird die Hydrierung in der Flüssigphase durchgeführt, so wird üblicherweise bei oder unterhalb einer Temperatur von 150°C gearbeitet, bevorzugterweise bei oder unterhalb von 100°C und in besonders bevorzugter Weise bei oder unterhalb von 80°C. Der anzuwendende Druck liegt bei der Durchführung der Hydrierung in der Gasphase üblicherweise oberhalb atmosphärischen Drucks, in bevorzugter Weise oberhalb 5 bar und in besonders bevorzugter Weise bei oder oberhalb von 10 bar sowie üblicherweise unterhalb von 50 bar, bevorzugterweise unterhalb von 40 bar und in besonders bevorzugter Weise bei oder unterhalb von 30 bar. Bei der Durchführung der Hydrierung in der Flüssigphase liegt der Druck üblicherweise oberhalb 5 bar, in bevorzugter Weise oberhalb 10 bar und in besonders bevorzugter Weise bei oder oberhalb von 15 bar sowie üblicherweise unterhalb von 100 bar, bevorzugterweise unterhalb von 80 bar und in besonders bevorzugter Weise bei oder unterhalb von 50 bar. Der Durchsatz durch den Reaktor, ausgedrückt als Raumgeschwindigkeit, beträgt bei der Durchführung der Hydrierung in der Gasphase üblicherweise mehr als 100 h⁻¹, in beispielsweise mehr als 500 h⁻¹ und in bevorzugter Weise 1000 h⁻¹ oder mehr sowie üblicherweise weniger als 10000 h⁻¹, beispielsweise weniger als 6000 h⁻¹ und in bevorzugter Weise 4000 h⁻¹ oder weniger. Bei der Durchführung der Hydrierung in der Flüssigphase beträgt der Durchsatz durch den Reaktor, ausgedrückt als Raumgeschwindigkeit, üblicherweise mindestens 0,1 h⁻¹, beispielsweise mehr als 0,5 h⁻¹ und in bevorzugter Weise 1 h⁻¹ oder mehr sowie üblicherweise höchstens 20 h⁻¹, beispielsweise weniger als 10 h⁻¹ und in bevorzugter Weise 5 h⁻¹ oder weniger. Wie bei praktisch allen Hydrierkatalysatoren ist ein gewisser langsamer Aufbau von Koksablagerungen auf dem Katalysator möglich, der jedoch durch periodisches Entkoken durch Behandlung des Katalysators mit Wasserdampf und Luft in dem Fachmann bekannter Weise entfernt werden kann. Im allgemeinen sind Zykluslängen von 1 bis 2 Jahren bei der Durchführung der Hydrierung in der Gasphase und von 2 bis 3 Jahren bei der Durchführung der Hydrierung in der Flüssigphase erreichbar. Die Gesamtlebensdauer des Katalysators kann dabei 5 bis 10 Jahre betragen.

In der zweiten Verfahrensstufe werden aus dem Stoffstrom Schwefel, Arsen und/oder Antimon enthaltende Verbindungen und gegebenenfalls restlicher Sauerstoff entfernt. Dazu wird der Stoffstrom nach seiner Behandlung in der der ersten Verfahrensstufe in einem Reaktor über eine Kontaktmasse geleitet, die Schwefel, Arsen und Antimon enthaltende Verbindungen absorbiert sowie Sauerstoff absorbiert und gegebenenfalls Sauerstoff durch Katalyse seiner Reaktion mit Kohlenmonoxid und/oder Wasserstoff aus dem Stoffstrom entfernt. Dies kann beispielsweise unter Verwendung der dem Fachmann bekannten, von Artrip et al., l. c., beschriebenen, Kupfer- und Zinkoxide enthaltenden Kontaktmassen, sofern diese zusätzlich noch metallisches Kupfer enthalten, geschehen. Diese Kontaktmassen können zusätzlich weitere Metalle oder Metalloxide enthalten, und sie können einen inerten Träger, beispielsweise ein anorganisches Oxid wie Aluminiumoxid oder Siliciumdioxid, umfassen. In bevorzugter Weise wird eine Kontaktmasse verwendet, die ein Gemisch von metallischem und oxidischem Kupfer, Zinkoxid und Aluminiumoxid darstellt.

Eine sehr gut geeignete Kontaktmasse besteht beispielsweise aus einem Gemisch von 30 bis 50 Gew.-% Kupferoxid, 30 bis 50 Gew.-% Zinkoxid und 0 bis 40 Gew.-% Aluminiumoxid, wobei sich die einzelnen Anteile stets zu 100 Gew.-% addieren, und wobei das Kupferoxid vor dem Einsatz der Kontaktmasse im Verfahren durch Behandlung mit einem Wasserstoff enthaltenden Gas weitgehend in metallisches Kupfer überführt wird.

Diese Kontaktmasse kann beispielsweise durch Fällung aus einer Lösung der entsprechenden Salze, Trocknung und Kalzination erhalten werden.

Bei der Verwendung solcher Kontaktmassen werden nicht nur Schwefel, Arsen und Antimon enthaltende Verbindungen absorbiert, sondern auch eventuell in der ersten Verfahrensstufe nicht zu Wasser hydrierter, überschüssiger Sauerstoff. Dieser Sauerstoff reagiert mit überschüssigem, in der ersten Verfahrensstufe nicht vollständig umgesetztem Wasserstoff und/oder als Verunreinigung vorhandenem Kohlenmonoxid unter Bildung von Wasser und/oder Kohlendioxid. Diese beiden Reaktionen werden von metallischem Kupfer katalysiert. Überschüssiger Sauerstoff, der mangels einer ausreichenden Kohlenmonoxid- oder Wasserstoffmenge nicht katalytisch entfernt werden kann, wird vom metallischen Kupfer unter Bildung von Kupferoxid absorbiert.

Die zweite Verfahrensstufe kann entweder als Gasphasenreaktion oder als Flüssigphasenreaktion durchgeführt werden. Die in der zweiten Verfahrensstufe anzuwendenden Reaktionsbedingungen hängen im wesentlichen von den vorhandenen Verunreinigungen ab.

Wenn ausschließlich Schwefel, Arsen und Antimon enthaltende Verunreinigungen zu absorbieren sind, wird sowohl bei einer Durchführung des Verfahrens in der Gasphase als auch in der Flüssigphase im allgemeinen eine Temperatur von mindestens 0°C, beispielsweise von 10°C oder mehr und in bevorzugter Weise von 15°C oder mehr sowie von höchstens 200°C, beispielsweise 150°C oder weniger und in bevorzugter Weise von 100°C oder weniger angewendet.

Wird die Reaktion in der Gasphase durchgeführt, so wird üblicherweise ein Druck von mindestens 0,1 bar (absolut), beispielsweise ein Druck von 0,5 bar (absolut) oder mehr und in bevorzugter Weise atmosphärischer oder höherer Druck sowie höchstens ein Druck von 200 bar, beispielsweise 150 bar oder weniger und in bevorzugter Weise 100 bar oder weniger angewendet.

Bei einer Durchführung der Reaktion in der Flüssigphase wird üblicherweise mindestens atmosphärischer Druck, beispielsweise ein Druck von 5 bar oder mehr und in bevorzugter Weise von 10 bar oder mehr sowie höchstens ein Druck von 200 bar, beispielsweise 150 bar oder weniger und in bevorzugter Weise 100 bar oder weniger angewendet.

Der Durchsatz durch den Reaktor, ausgedrückt als Raumgeschwindigkeit, beträgt bei der Durchführung der Reaktion in der Gasphase üblicherweise mindestens 500 h⁻¹, beispielsweise 1000 h⁻¹ oder mehr und in bevorzugter Weise 2000 h⁻¹ oder mehr sowie üblicherweise höchstens 10000 h⁻¹, beispielsweise 5000 h⁻¹ oder weniger und in bevorzugter Weise 3000 h⁻¹ oder weniger.

Bei der Durchführung der Reaktion in der Flüssigphase beträgt der Durchsatz durch den Reaktor, ausgedrückt als Raumgeschwindigkeit, üblicherweise mindestens 1 h⁻¹, beispielsweise 2 h⁻¹ oder mehr und in bevorzugter Weise 3 h⁻¹ oder mehr sowie üblicherweise höchstens 20 h⁻¹, beispielsweise 10 h⁻¹ oder weniger und in bevorzugter Weise 5 h⁻¹ oder weniger.

Typischerweise werden, abhängig vom Verunreinigungsgrad, von der eingesetzten Menge an Kontaktmasse und von den Betriebsbedingungen Betriebszeiten von 2 bis 5 Jahren erreicht, bevor die Absorptionskapazität der Kontaktmasse erschöpft ist.

Wenn der dem zweiten Verfahrensschritt zugeführte Stoffstrom noch nicht umgesetzten Sauerstoff als Verunreinigung mit sich führt, ist die Wahl der Verfahrensparameter von weiteren Bedingungen abhängig. Wenn zusätzlich zum Sauerstoff noch in der ersten verfahrensstufe nicht umgesetzter Wasserstoff im Stoffstrom vorhanden ist, so beträgt sowohl bei der Durchführung der Reaktion in der Gasphase als auch bei ihrer Durchführung in der Flüssigphase statt der oben genannten unteren Grenze des Temperaturbereichs die Reaktionstemperatur mindestens 50°C, beispielsweise 60°C oder mehr und in bevorzugter Weise 70°C oder mehr. Ist im Stoffstrom Kohlenmonoxid als verunreinigung enthalten, so beträgt sowohl bei der Durchführung der Reaktion in der Gasphase als auch bei ihrer Durchführung in der Flüssigphase statt der oben genannten unteren Grenze des Temperaturbereichs die Reaktionstemperatur mindestens 15°C, beispielsweise 30°C oder mehr und in bevorzugter Weise 40°C oder mehr.

Bei der Durchführung der Reaktion ist darauf zu achten, daß in der Kontaktmasse stets Kupfer im metallischen Zustand vorliegt. Wenn im Stoffstrom, bezogen auf seinen Sauerstoffgehalt, ein stöchiometrischer Überschuß an Wasserstoff und/oder Kohlenmonoxid vorliegt, wird über die durch die Absorptionskapazität der Kontaktmasse bestimmte Betriebsdauer auch stets ein Anteil metallisches Kupfer vorhanden sein. Liegt jedoch ein stöchiometrischer Überschuß von Sauerstoff vor, so muß die Kontaktmasse spätestens beim Auftreten von Sauerstoff im Produkt der zweiten Verfahrensstufe durch Behandlung mit einem Wasserstoff und/oder Kohlenmonoxid enthaltenden Gas regeneriert werden. Die Betriebsdauern bis zu einer notwendigen Regenerierung sind in diesem Fall vom Sauerstoffgehalt des Stoffstroms und von den Betriebsbedingungen, insbesondere von der Temperatur, abhängig und betragen in der Regel einige Monate bis zu einem Jahr. Regenerierungsverfahren für metallisches Kupfer enthaltende Katalysatoren, die durch Sauerstoff oxidiert wurden, sind dem Fachmann bekannt und können auch für die Regenerierung der im zweiten Verfahrensschritt des erfindungsgemäßen Verfahrens vorzugsweise eingesetzten Kontaktmasse angewendet werden.

Wenn die Reaktion in der Gasphase durchgeführt wird, beträgt die Temperatur mindestens 0°C, beispielsweise 10°C oder mehr und in bevorzugter Weise 20°C oder mehr sowie höchstens 200°C, beispielsweise 150°C oder weniger und in bevorzugter Weise 130°C oder weniger. Der anzuwendende Druck ist mindestens 0,1 bar (absolut), beispielsweise 0,5 bar (absolut) oder mehr und in bevorzugter Weise atmosphärischer oder höherer Druck sowie höchstens 200 bar, beispielsweise 150 bar oder weniger und in bevorzugter Weise 100 bar oder weniger. Der Durchsatz durch den Reaktor, ausgedrückt als Raumgeschwindigkeit, beträgt normalerweise mindestens 100 h⁻¹, beispielsweise 500 h⁻¹ oder mehr und in bevorzugter Weise 1000 h⁻¹ oder mehr sowie üblicherweise höchstens 10000 h⁻¹, beispielsweise 5000 h⁻¹ oder weniger und in bevorzugter Weise 2500 h⁻¹ oder weniger.

Bei einer Durchführung der Reaktion in der Flüssigphase liegt die Temperatur üblicherweise bei mindestens 0°C, beispielsweise bei 10°C oder mehr und in bevorzugter Weise bei 20°C oder mehr sowie höchstens bei 150°C, beispielsweise bei 100°C oder weniger und in bevorzugter Weise bei 80°C oder weniger. Der anzuwendende Druck ist mindestens atmosphärischer Druck, beispielsweise 5 bar oder mehr und in bevorzugter Weise 10 bar oder mehr sowie höchstens 200 bar, beispielsweise 150 bar oder weniger und in bevorzugter Weise 100 bar oder weniger. Der Durchsatz durch den Reaktor, ausgedrückt als Raumgeschwindigkeit, beträgt mindestens 0,1 h⁻¹, beispielsweise 0,5 h⁻¹ oder mehr und in bevorzugter Weise 1 h⁻¹ oder mehr sowie üblicherweise höchstens 15 h⁻¹, beispielsweise 10 h⁻¹ oder weniger und in bevorzugter Weise 5 h⁻¹ oder weniger.

Wenn zusätzlich zum Sauerstoff noch in der ersten Verfahrensstufe nicht umgesetzter Wasserstoff im Stoffstrom vorhanden ist, so beträgt sowohl bei der Durchführung der Reaktion in der Gasphase als auch bei ihrer Durchführung in der Flüssigphase statt der oben genannten unteren Grenze des Temperaturbereichs die Reaktionstemperatur mindestens 50°C, beispielsweise 60°C oder mehr und in bevorzugter Weise 70°C oder mehr. Ist im Stoffstrom Kohlenmonoxid als Verunreinigung enthalten, so beträgt sowohl bei der Durchführung der Reaktion in der Gasphase als auch bei ihrer Durchführung in der Flüssigphase statt der oben genannten unteren Grenze des Temperaturbereichs die Reaktionstemperatur mindestens 15°C, beispielsweise 30°C oder mehr und in bevorzugter Weise 40°C oder mehr.

In der dritten Verfahrensstufe werden überschüssiges Kohlenmonoxid und überschüssiger Wasserstoff, die bei einem stöchiometrischen Sauerstoffunterschuß in der zweiten Verfahrensstufe im Produkt dieser zweiten Verfahrensstufe noch enthalten sind, entfernt. Die Durchführung einer dritten Verfahrensstufe ist nicht in allen Fällen notwendig. Eine dritte Verfahrensstufe zur Entfernung von Kohlenmonoxid und Wasserstoff ist beispielsweise dann unnötig, wenn Kohlenmonoxid und Wasserstoff bereits in der zweiten Verfahrensstufe mit Sauerstoff vollständig umgesetzt wurde.

Der zu reinigende Stoffstrom wird in der dritten Verfahrensstufe, sofern erforderlich, zur Entfernung von restlichen Verunreinigungen durch Kohlenmonoxid und Wasserstoff über eine Kontaktmasse geleitet, die Kohlenmonoxid und/oder Wasserstoff aus dem Stoffstrom absorptiv und/oder durch chemische Reaktionen entfernt. Dies kann beispielsweise unter Verwendung der dem Fachmann bekannten, von Artrip et al., l. c., beschriebenen, Kupfer- und Zinkoxide enthaltenden Kontaktmassen geschehen. Diese Kontaktmassen können zusätzlich weitere Metalle oder Metalloxide enthalten, und sie können einen inerten Träger, beispielsweise ein anorganisches Oxid wie Aluminiumoxid oder Siliciumdioxid, umfassen. In bevorzugter Weise wird eine Kontaktmasse verwendet, die ein Gemisch von oxidischem Kupfer, Zinkoxid und Aluminiumoxid darstellt.

Eine sehr gut geeignete Kontaktmasse besteht beispielsweise aus einem Gemisch von 30 bis 50 Gew.-% Kupferoxid, 30 bis 50 Gew.-% Zinkoxid und 0 bis 40 Gew.-% Aluminiumoxid, wobei sich die einzelnen Anteile stets zu 100 Gew.-% addieren.

Diese Kontaktmasse kann beispielsweise durch Fällung aus einer Lösung der entsprechenden Salze, Trocknung und Kalzination erhalten werden.

Die beschriebene Kontaktmasse reagiert mit Kohlenmonoxid unter Bildung von metallischem Kupfer und Kohlendioxid sowie mit Wasserstoff unter Bildung von metallischem Kupfer und Wasser. Beim Betrieb wird also das in der Kontaktmasse enthaltene Kupferoxid allmählich in metallisches Kupfer umgewandelt und die Kapazität der Kontaktmasse für die Umwandlung von Kohlenmonoxid und Wasserstoff zu Kohlendioxid und Wasser erschöpft sich im Laufe der Zeit. In der zweiten und in der dritten Verfahrensstufe können im im Prinzip identische Kontaktmassen eingesetzt werden, die sich nur dadurch unterscheiden, daß die Kontaktmasse der zweiten Verfahrensstufe zu Betriebsbeginn einen Anteil metallisches Kupfer enthält, der bei einem stöchiometrischen Sauerstoffüberschuß relativ zum Kohlenmonoxid- und Wasserstoffgehalt im Stoffstrom allmählich in Kupferoxid umgewandelt wird, während die Kontaktmasse der dritten Verfahrensstufe zu Betriebsbeginn kein metallisches Kupfer enthält, dieses aber durch Reaktion mit Kohlenmonoxid und Wasserstoff allmählich in metallisches Kupfer umgewandelt wird. Es kann also dieselbe Kontaktmasse, einmal im teilweise reduzierten Zustand und einmal im oxidierten Zustand, sowohl für die zweite als auch für die dritte Verfahrensstufe eingesetzt werden. Derartige Kontaktmassen sind kommerziell erhältlich und können beispielsweise unter der Bezeichnung R3-15 bei BASF Aktiengesellschaft, Ludwigshafen, Deutschland, bezogen werden.

Die Durchführung der dritten Verfahrensstufe ist sowohl als Gas-phasenreaktion wie auch als Flüssigphasenreaktion möglich.

Wenn die Reaktion in der Gasphase durchgeführt wird, beträgt die Temperatur mindestens 50°C, beispielsweise 80°C oder mehr und in bevorzugter Weise 110°C oder mehr sowie höchstens 200°C, beispielsweise 150°C oder weniger und in bevorzugter Weise 130°C oder weniger. Der anzuwendende Druck ist mindestens 0,1 bar (absolut), beispielsweise 0,5 bar (absolut) oder mehr und in bevorzugter Weise atmosphärischer oder höherer Druck sowie höchstens 200 bar, beispielsweise 150 bar oder weniger und in bevorzugter Weise 100 bar oder weniger. Der Durchsatz durch den Reaktor, ausgedrückt als Raumgeschwindigkeit, beträgt mindestens 100 h⁻¹, beispielsweise 500 h⁻¹ oder mehr und in bevorzugter Weise 1000 h⁻¹ oder mehr sowie üblicherweise höchstens 10000 h⁻¹, beispielsweise 5000 h⁻¹ oder weniger und in bevorzugter Weise 2500 h⁻¹ oder weniger.

Bei einer Durchführung der Reaktion in der Flüssigphase liegt die Temperatur üblicherweise bei mindestens 50°C, beispielsweise bei 80°C oder mehr und in bevorzugter Weise bei 110°C oder mehr sowie höchstens bei 250°C, beispielsweise bei 200°C oder weniger und in bevorzugter Weise bei 150°C oder weniger. Der anzuwendende Druck ist mindestens atmosphärischer Druck, beispielsweise 5 bar oder mehr und in bevorzugter Weise 10 bar oder mehr sowie höchstens 200 bar, beispielsweise 150 bar oder weniger und in bevorzugter Weise 100 bar oder weniger. Der Durchsatz durch den Reaktor, ausgedrückt als Raumgeschwindigkeit, beträgt mindestens 0,1 h⁻¹, beispielsweise 0,5 h⁻¹ oder mehr und in bevorzugter Weise 1 h⁻¹ oder mehr sowie üblicherweise höchstens 15 h⁻¹, beispielsweise 10 h⁻¹ oder weniger und in bevorzugter Weise 5 h⁻¹ oder weniger.

Sobald im Produkt der dritten Verfahrensstufe Kohlenmonoxid und/oder Wasserstoff als Verunreinigungen auftreten, muß die Kontaktmasse durch Behandlung mit einem Sauerstoff enthaltenden Gas regeneriert werden. Derartige Regenerierungsverfahren für zu metallischem Kupfer reduzierte Kupferoxid-Kontaktmassen sind dem Fachmann bekannt. Die erreichbaren Zyklusdauern zwischen den Regenerierungen sind abhängig vom Verunreinigungsgrad des zu reinigende Stoffstroms, von den Verfahrensbedingungen und der eingesetzten Menge an Kontaktmasse. Typischerweise können Zyklusdauern bis zu 2 Jahren erreicht werden.

Das erfindungsgemäße Verfahren vermeidet die Nachteile der bekannten Verfahren, insbesondere massive Fouling-Probleme oder die Möglichkeit einer Kupferacetylid-Bildung. Mit höchstens drei Verfahrensstufen ist es darüber hinaus wirtschaftlicher als die bekannten Verfahren.

Das erfindungsgemäße Verfahren kann allgemein zur Reinigung von Stoffströmen, die durch Hydrierung entfernbare Verunreinigungen enthalten und auch durch Schwefel, Arsen und/oder Antimon enthaltende Verbindungen, Kohlenmonoxid und/oder Sauerstoff verunreinigt sein können, eingesetzt werden. Besonders vorteilhaft anwendbar ist es zur Reinigung von Olefinströmen von Verunreinigungen durch Alkine und/oder Diene und/oder Sauerstoff in einer ersten Verfahrensstufe, von Verunreinigungen durch Schwefel, Arsen und/oder Antimon enthaltende Verbindungen sowie Kohlenmonoxid und/oder restlichen Sauerstoff und/oder restlichen Wasserstoff in einer zweiten Verfahrensstufe und gegebenenfalls von Verunreinigungen durch restliches Kohlenmonoxid und/oder restlichen Wasserstoff in einer dritten Verfahrensstufe.

Stoffströme, aus denen mit dem erfindungsgemäßen Verfahren die genannten Verunreinigungen entfernt worden sind, enthalten Anteile an im Verlauf des erfindungsgemäßen Verfahrens entstandenen Nebenprodukten, nämlich Kohlendioxid, Wasser und/oder Alkanen. Diese Nebenprodukte verhalten sich in weiteren Reaktionen, denen die gereinigten Stoffströme unterworfen werden, im allgemeinen inert. Falls erforderlich oder gewünscht, können diese Nebenprodukte jedoch auch nach der Durchführung des erfindungsgemäßen Verfahrens aus dem gereinigten Stoffstrom auf dem Fachmann bekannte Weise, beispielsweise durch Absorption an mikroporösen Feststoffen, abgetrennt werden.

## Patentansprüche

1. Verfahren zur Entfernung von Alkinen, Sauerstoff, Schwefel, Arsen und/oder Antimon enthalten, wahlweise auch Kohlenmonoxid und wahlweise auch Dienen oder Dienen und einfach ungesättigten Kohlenwasserstoffen aus Stoffströmen, die Alkine, Sauerstoff, Verbindungen, die Schwefel, Arsen und/oder Antimon enthalten, und Kohlenmonoxid umfassen, **dadurch gekennzeichnet, daß** man höchstens drei Verfahrensstufen anwendet, wobei man in der genannten Reihenfolge:
• in einer ersten Verfahrensstufe nach Zugabe von Wasserstoff zum Einsatzstoffstrom an einem Silber und Palladium enthaltenden Hydrierkatalysator mit einem Siliciumdioxid enthaltenden anorganischen oxidischen Träger Alkine, zumindest teilweise auch Sauerstoff, und wahlweise auch Diene oder Diene und einfach ungesättigte Kohlenwasserstoffe durch Hydrierung entfernt;
• restlichen Sauerstoff aus der ersten Verfahrensstufe und Schwefel, Arsen und/oder Antimon enthaltende Verbindungen in einer zweiten Verfahrensstufe an einer Kontaktmasse durch Absorption abreichert oder entfernt und gegebenenfalls restlichen Sauerstoff mit Kohlenmonoxid und/oder restlichem Wasserstoff zumindest teilweise an dieser Kontaktmasse katalytisch zu Wasser und Kohlendioxid umsetzt;
• gegebenenfalls in einer dritten Verfahrensstufe restliches Kohlenmonoxid und/oder restlichen Wasserstoff aus den vorangegangenen Verfahrensstufen an einer Kontaktmasse in Kohlendioxid und/oder Wasser umwandelt.

2. Verfahren nach Anspruch 1, **dadurch gekennzeichnet, daß** der Stoffstrom ein Olefinstrom ist.

3. Verfahren nach Anspruch 1, **dadurch gekennzeichnet, daß** der Siliciumdioxid enthaltende anorganische Träger Kieselgur ist.

## Claims

1. A process for removing alkynes, oxygen, compounds which contain sulfur, arsenic and/or antimony, optionally also carbon monoxide and optionally also dienes or dienes and monounsaturated hydrocarbons from material streams which comprise alkynes, oxygen, compounds which contain sulfur, arsenic and/or antimony, and carbon monoxide, wherein not more than three process stages are used, in the stated sequence:
• in a first process stage, after addition of hydrogen to the stream of starting materials, alkynes, at least partly also oxygen, and optionally also dienes or dienes and monounsaturated hydrocarbons are removed by hydrogenation over the silver- and palladium-containing hydrogenation catalyst having a silica-containing inorganic oxidic support;
• residual oxygen from the first process stage and sulfur-, arsenic- and/or antimony-containing compounds are reduced in concentration or removed in a second process stage over a catalyst material by absorption and, if desired, residual oxygen with carbon monoxide and/or residual hydrogen are at least partially catalytically converted over this catalyst material into water and carbon dioxide;
• if required, in a third process stage, residual carbon monoxide and/or residual hydrogen from the preceding process stages are converted into carbon dioxide and/or water over a catalyst material.

2. A process as claimed in claim 1, wherein the material stream is an olefin stream.

3. A process as claimed in claim 1, wherein the silica-containing inorganic support is kieselguhr.

## Revendications

1. Procédé pour l'élimination d'alcynes, d'oxygènes, de composés qui contiennent du soufre, de l'arsenic et/ou de l'antimoine, sélectivement aussi du monoxyde de carbone et sélectivement aussi des diènes ou des diènes et des hydrocarbures monoinsaturés dans des écoulements de matière qui contiennent des alcynes, de l'oxygène, des composés qui contiennent du soufre, de l'arsenic et/ou de l'antimoine ainsi que du monoxyde de carbone, **caractérisé en ce que** l'on utilise au plus trois étapes de traitement par lesquelles on fait réagir dans la succession indiquée:
- dans une première étape de traitement, après addition d'hydrogène à l'écoulement de matières premières, on élimine les alcynes, au moins en partie également l'oxygène et sélectivement également des diènes ou des diènes et des hydrocarbures monoinsaturés par hydrogénation sur un catalyseur d'hydrogénation contenant de l'argent et du palladium avec un support oxydé minéral contenant du dioxyde de silicium;
- dans une deuxième étape de traitement, on appauvrit ou on élimine par absorption l'oxygène résiduel provenant de la première étape de traitement ainsi que des composés contenant du soufre, de l'arsenic et/ou de l'antimoine, et on convertit éventuellement, au moins en partie catalytiquement, l'oxygène résiduel avec du monoxyde de carbone et/ou l'hydrogène résiduel pour obtenir de l'eau et du dioxyde de carbone;
- éventuellement, dans une troisième étape de traitement, on convertit le monoxyde de carbone résiduel et/ou l'hydrogène résiduel provenant des étapes de traitement précédentes en dioxyde de carbone et/ou en eau sur une pâte de contact.

2. Procédé selon la revendication 1, **caractérisé en ce que** le flux de matière est un flux d'oléfine.

3. Procédé selon la revendication 1, **caractérisé en ce que** le support minéral contenant du dioxyde de silicium est constitué de diatomite.
